# EUROPEAN PATENT SPECIFICATION

(11) **EP 1 974 670 B1**
(45) Date of publication and mention of the grant of the patent: **12.05.2010**
(21) Application number: 08004985.1
(22) Date of filing: 17.03.2008
(51) Int. Cl.: A61B 8/00

(54) **Ultrasonic observation apparatus and ultrasonic diagnostic apparatus**
Ultraschallbeobachtungsgerät und Ultraschalldiagnosegerät
Appareil d'observation ultrasonique et appareil de diagnostic ultrasonique

(30) Priority: 29.03.2007 JP 2007089014
(43) Date of publication of application: 01.10.2008
(73) Proprietor: Olympus Medical Systems Corp., Tokyo 151-0072 (JP)
(72) Inventor: Ichikawa, Junichi, Tokyo 151-0072 (JP)
(74) Representative: von Hellfeld, Axel

(56) References cited:
- EP-A- 1 629 779
- WO-A-00/66002
- WO-A-2005/053664
- US-A- 5 279 301
- US-A1- 2004 002 657

## Description

### BACKGROUND OF THE INVENTION

### 1. Field of the Invention

The present invention relates to an ultrasonic observation apparatus capable of connecting an electron scanning ultrasonic probe and mechanical scanning ultrasonic probe as well as to an ultrasonic diagnostic apparatus using the ultrasonic observation apparatus.

### 2. Description of the Related Art

Conventionally, ultrasonic diagnostic apparatus transmit ultrasonic pulses repeatedly to body tissue from an ultrasonic transducer of an ultrasonic probe connected to an ultrasonic observation apparatus, receive an echo signal of the ultrasonic pulses reflected by the body tissue, generate visible images--ultrasonic tomographic images--from information contained in a living body, and display the ultrasonic tomographic images on a display unit such as a monitor.

Various types of ultrasonic probes are connected to the ultrasonic observation apparatus of such an ultrasonic diagnostic apparatus according to observed sites and diagnostic uses. For example, the ultrasonic observation apparatus may be detachably connected with both electron scanning ultrasonic probe and mechanical scanning ultrasonic probe.

The electron scanning ultrasonic probe is an ultrasonic probe of an electron scanning type which scans an inside of a body cavity by electronically driving an internal ultrasonic transducer. On the other hand, the mechanical scanning ultrasonic probe is an ultrasonic probe of a mechanical scanning type which scans an inside of a body cavity by mechanically rotating an internal ultrasonic transducer.

Since multiple types of ultrasonic probes are connected to the ultrasonic observation apparatus according to observed sites and diagnosis in this way, various setting parameters related to operation control of the ultrasonic probe and generation of ultrasonic images need to be changed according to the type of ultrasonic probe connected to the ultrasonic observation apparatus.

Conventional techniques which change various setting parameters of the ultrasonic observation apparatus according to the type of connected ultrasonic probe include ultrasonic diagnostic apparatuses disclosed in Japanese Patent Laid-Open Nos. 7-299065, 2005-230379, and 2007-14485.

To provide expandability so as not to limit types of connectable probe, Japanese Patent Laid-Open No. 7-299065 described above discloses a technique related to an ultrasonic diagnostic apparatus which controls programs and parameters for frequencies and focusing methods of transmitted ultrasound as well as programs and parameters for providing guidelines and displaying frequencies during image display based on the connected ultrasonic probe.

Also, Japanese Patent Laid-Open No. 2005-230379 described above discloses a technique related to a connection adaptor for an ultrasonic inspection apparatus. The connection adaptor allows a mechanical scanning ultrasonic probe to be connected to the ultrasonic observation apparatus to carry out mechanical ultrasonic scanning, as required, by connecting the mechanical scanning ultrasonic probe to the ultrasonic observation apparatus connected with an electron scanning ultrasonic probe. Also, the connection adaptor is designed to change scan origin position and frame rate setting based on the type of connected ultrasonic probe.

Furthermore, Japanese Patent Laid-Open No. 2007-14485 discloses a technique related to an ultrasonic observation apparatus designed to change display format of ultrasonic images and various setting parameters based on the type of connected ultrasonic probe to make it easy to add new ultrasonic probes without increasing man-hours.

The ultrasonic observation apparatus used for the conventional ultrasonic diagnostic apparatus transmits ultrasonic pulses repeatedly from an ultrasonic transducer of a connected electron scanning ultrasonic probe or mechanical scanning ultrasonic probe to body tissue and receives an echo signal of the ultrasonic pulses reflected by the body tissue.

Based on sound ray data contained in luminance information obtained from the received echo signal, the ultrasonic observation apparatus performs a coordinate transformation using a coordinate transformation table to generate pixel data at pixel locations for display on a monitor screen and thereby generates an ultrasonic image in a frame memory.

However, if an electron scanning ultrasonic probe is connected to the ultrasonic observation apparatus, pixel count in an item of sound ray data obtained by the electron scanning ultrasonic probe differs between B mode and flow mode (also known as Doppler mode). Consequently, if the type of electron scanning ultrasonic probe or a range of the electron scanning ultrasonic probe is changed, it is not possible to perform a coordinate transformation using the same coordinate transformation table as before the replacement or range changing of the ultrasonic probe to generate pixel data at pixel locations for display on the monitor screen.

Therefore, with the conventional ultrasonic observation apparatus, multiple coordinate transformation tables are prepared to accommodate different types or ranges of electron scanning ultrasonic probes and the coordinate transformation table for use in coordinate transformation is switched according to the type and range of the connected ultrasonic probe.

This increases amounts of memory required to store the coordinate transformation tables, resulting in increased cost. Also, an electron scanning ultrasonic probe cannot be connected to the ultrasonic observation apparatus if an appropriate coordinate transformation table is not available.

On the other hand, if a mechanical scanning ultrasonic probe is connected to the ultrasonic observation apparatus, conventionally sound ray data with appropriate pixel count per data item is obtained in each of modes (e.g., two B modes for 512-pixel and 1024-pixel monochrome images) preset on the mechanical scanning ultrasonic probe. Consequently, two coordinate transformation tables are needed to generate pixel data at pixel locations for display in the two modes which support different pixel counts.

That is, whichever of the electron scanning ultrasonic probe and mechanical scanning ultrasonic probe may be used, if the coordinate transformation table is regenerated according to the type or range of the ultrasonic probe, coordinate transformation can be performed according to the type or range of the ultrasonic probe using only the single coordinate transformation table. This reduces memory requirements and costs. However, with the conventional ultrasonic observation apparatus, the coordinate transformation table cannot be regenerated according to the type or range of the ultrasonic probe.

Japanese Patent Laid-Open Nos. 7-299065 , 2005-230379 , and 2007-14485 described above disclose techniques for changing various setting parameters of the ultrasonic observation apparatus according to the type of connected ultrasonic probe, but do not disclose or suggest anything about concrete components, namely, concrete means and methods for regenerating the coordinate transformation table according to the type or range of the ultrasonic probe.

Document WO 2005/053664 A2 concerns a modular portable ultrasound system in which different kinds of ultrasound transducers may be attached via an integral data/video cable to a portable data/video processing unit. The portable data/video processing unit is capable of identifying the type of ultrasound transducer attached to the portable data/video processing unit. Pre-stored application data is loaded from a hard disk depending on the determined transducer type.

Document WO 00/66002 concerns an ultrasound diagnostic instrument having a memory in a detachable transducer scan head that is separate from a console of the ultrasound diagnostic instrument, whereby the memory stores operation data unique to the specific transducer scan head.

Document US 2004/0002657 concerns an ultrasonic imaging system having a detachable scan head comprising a microcode. When the scan head is attached to the ultrasound imaging system, archives of the imaging system are searched based on the microcode included in the scan head for appropriate microcode. If a matching microcode is found, respective initialization data for the scan head is loaded into a volatile random access memory. In case no matching microcode is found, the operator of the ultrasound imaging system is alerted.

Document US 5,279,301 concerns an ultrasonic image analyzing apparatus which is capable of distinguishing the types of the ultrasonic probes attached to the ultrasonic image observation apparatus. For this, a scope identifying circuit is provided. Based on the output of the scope identifying circuit, parameters corresponding to the attached ultrasonic probe are obtained from a parameter storing circuit and used for the analyzing.

Document EP 1 629 779 A1 concerns an ultrasound probe diagnosing apparatus in which a control unit acquires focus information concerning an ultrasound probe connected via a connector to the apparatus by means of identification information stored in an identification information output unit of the ultrasound probe

The present invention has been made in view of the above circumstances and has an object to provide an ultrasonic observation apparatus capable of regenerating a coordinate transformation table according to a type or range of an ultrasonic probe as well as to provide an ultrasonic diagnostic apparatus which uses the ultrasonic observation apparatus.

### SUMMARY OF THE INVENTION

Some or all of the above problems are overcome by an ultrasonic observation apparatus having the features of claim 1.

The present invention provides an ultrasonic observation apparatus which generates an ultrasonic image from sound ray data obtained by a detachable ultrasonic probe, including: a coordinate transformation table for use to generate image data at pixel locations for display from the sound ray data obtained by the ultrasonic probe; and a control unit which detects a type or range of the detachable ultrasonic probe and regenerates the coordinate transformation table based on the detected type or range of the detachable ultrasonic probe.

Also, the present invention provides an ultrasonic diagnostic apparatus including: a detachable electron scanning ultrasonic probe; a detachable mechanical scanning ultrasonic probe; an ultrasonic observation apparatus which generates respective ultrasonic images from sound ray data obtained by the detachable electron scanning ultrasonic probe and the detachable mechanical scanning ultrasonic probe, the ultrasonic observation apparatus including a coordinate transformation table for use to generate image data at pixel locations for display from the sound ray data obtained by the detachable electron scanning ultrasonic probe and the detachable mechanical scanning ultrasonic probe, and a control unit which detects types or ranges of the detachable electron scanning ultrasonic probe and the detachable mechanical scanning ultrasonic probe and regenerates the coordinate transformation table based on the detected types or ranges of the detachable electron scanning ultrasonic probe and the detachable mechanical scanning ultrasonic probe; and a display unit which displays the ultrasonic images generated by the ultrasonic observation apparatus.

### BRIEF DESCRIPTION OF THE DRAWINGS

Fig. 1 is a block diagram showing an overall configuration of an ultrasonic diagnostic apparatus according to a first embodiment of the present invention;
Fig. 2 is a schematic diagram showing sound ray data obtained by an electron scanning ultrasonic probe in Fig. 1;
Fig. 3 is a schematic diagram showing sound ray data obtained by a mechanical scanning ultrasonic probe in Fig. 1;
Fig. 4 is a schematic diagram showing sound ray data obtained by an electron scanning ultrasonic probe different from the electron scanning ultrasonic probe in Fig. 2;
Fig. 5 is a diagram showing an example of a circular image displayed when the electron scanning ultrasonic probe in Fig. 2 is used;
Fig. 6 is a diagram showing an example of a fan-shaped image displayed when the mechanical scanning ultrasonic probe in Fig. 3 is used;
Fig. 7 is a diagram illustrating a display range of an ultrasonic image based on sound ray data of 512 pixels out of sound ray data of 1024 pixels on an entire circumference obtained by the electron scanning ultrasonic probe;
Fig. 8 is a schematic diagram of pixel data in a coordinate transformation table and frame memory, illustrating a coordinate transformation process of generating pixel data at pixel locations for display on a monitor screen from sound ray data shown in Fig. 7;
Fig. 9 is an enlarged explanatory diagram showing pixels based on any given item of sound ray data in Fig. 8 and pixels at pixel locations for display after coordinate transformation;
Fig. 10 is a flowchart showing an example of basic control performed by a control unit in Fig. 1;
Fig. 11 is a flowchart showing an example of control performed by the control unit when the ultrasonic probe in Fig. 1 is pulled out or a range of the ultrasonic probe is changed;
Fig. 12 is a diagram showing an example of dual-screen display of a three-dimensional ultrasonic image and two-dimensional ultrasonic image;
Fig. 13 is a diagram showing another example of dual-screen display of a three-dimensional ultrasonic image and two-dimensional ultrasonic image;
Fig. 14 is a diagram showing an example of display in three-dimensional ultrasonic image display mode;
Fig. 15 is a diagram showing a variation of the three-dimensional ultrasonic image display mode in Fig. 14;
Fig. 16 is a diagram showing a display example of how to measure a volume by specifying three points at an observed site on a displayed three-dimensional ultrasonic image;
Fig. 17 is a diagram showing a display example of how volume measurements are allowed to be taken twice at an observed site on a three-dimensional ultrasonic image;
Fig. 18 is a diagram showing a display example of a normal two-dimensional ultrasonic image on a display screen;
Fig. 19 is a diagram showing a variation of dual-screen display mode of the ultrasonic observation apparatus;
Fig. 20 is a diagram showing an example of display on a display screen to illustrate capabilities to display thumbnails by reading out prestored ultrasonic images; and
Fig. 21 is a perspective block diagram showing an external configuration of an ultrasonic observation apparatus equipped with an active switch.

### DETAILED DESCRIPTION OF THE PREFERRED EMBODIMENTS

Embodiments of the present invention will be described below with reference to the drawings.

### (First embodiment)

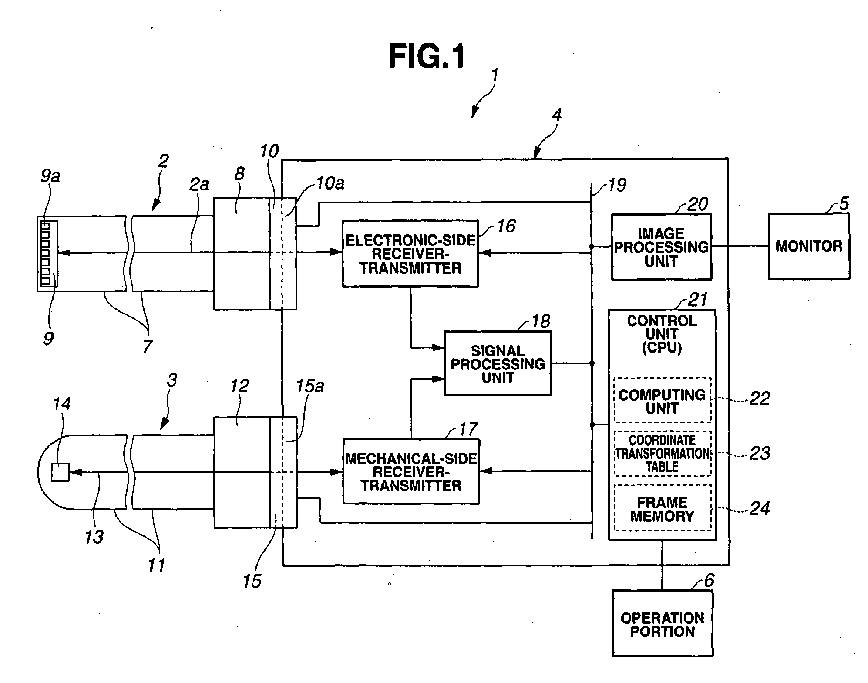

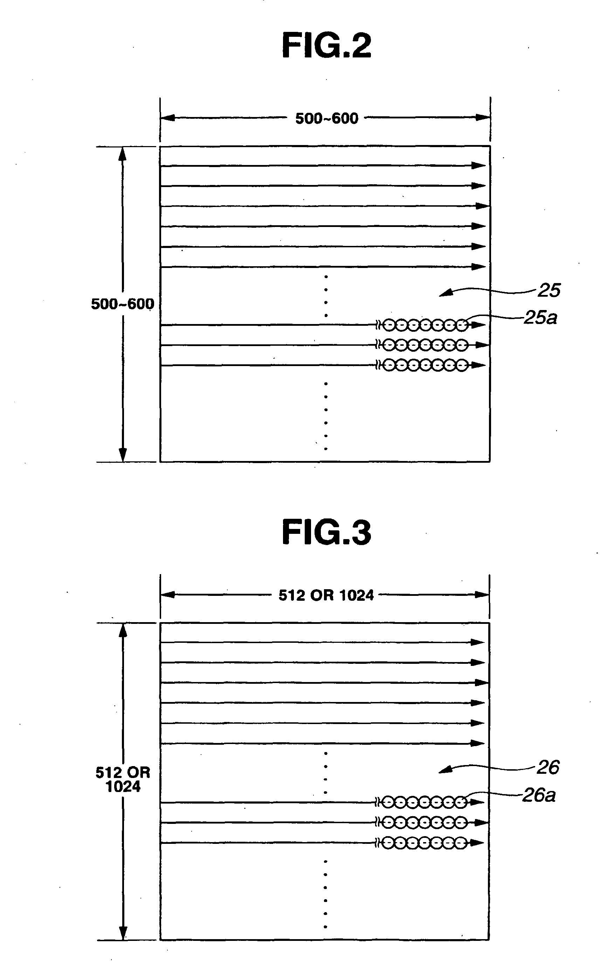

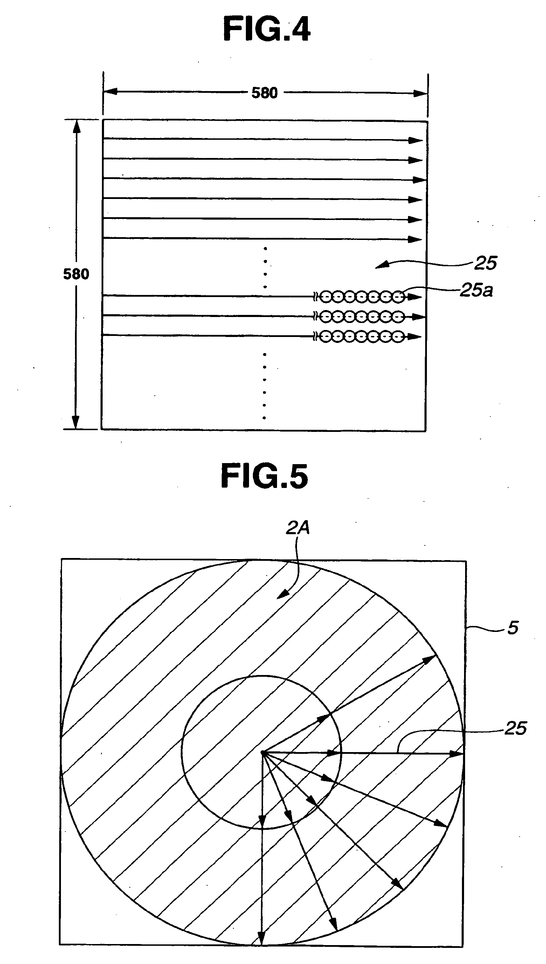

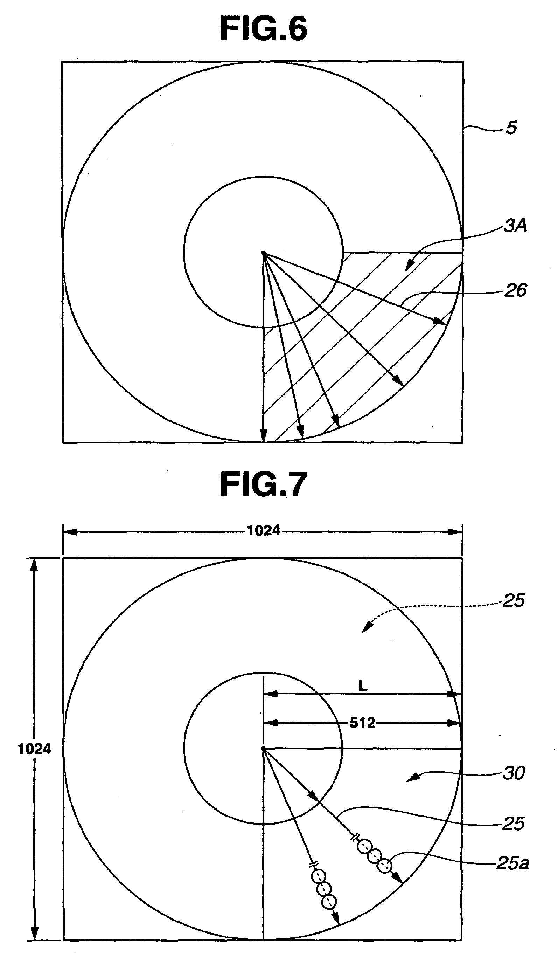

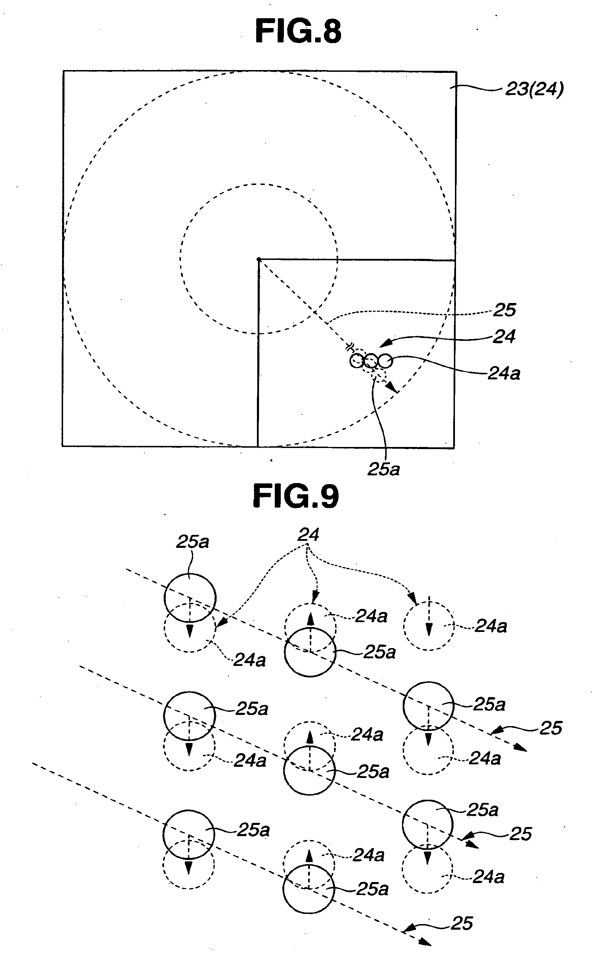

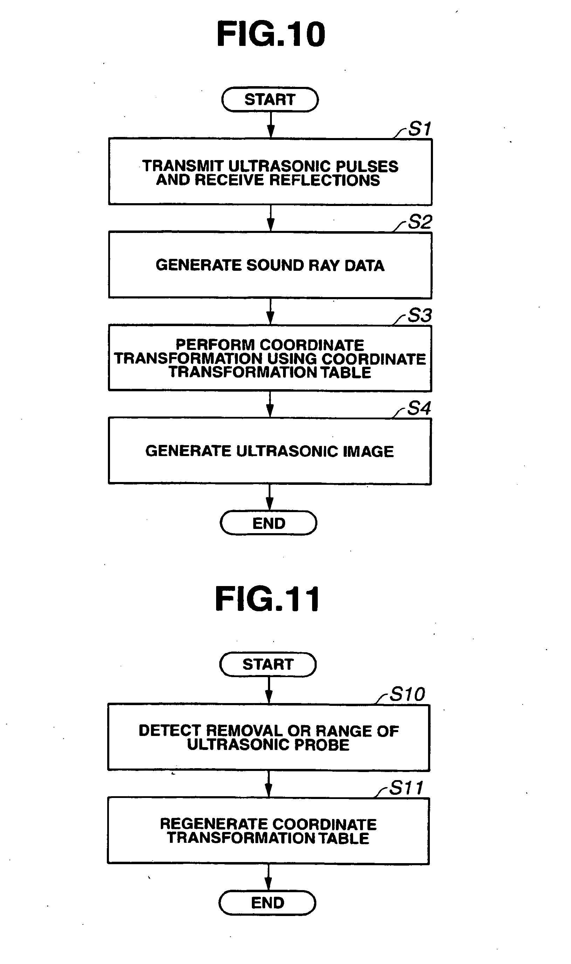

Figs. 1 to 11 concern a first embodiment of the present invention, where Fig. 1 is a block diagram showing an overall configuration of an ultrasonic diagnostic apparatus according to the first embodiment, Fig. 2 is a schematic diagram showing sound ray data obtained by an electron scanning ultrasonic probe in Fig. 1, Fig. 3 is a schematic diagram showing sound ray data obtained by a mechanical scanning ultrasonic probe in Fig. 1, Fig. 4 is a schematic diagram showing sound ray data obtained by an electron scanning ultrasonic probe different from the electron scanning ultrasonic probe in Fig. 2, Fig. 5 is a diagram showing an example of a circular image displayed when the electron scanning ultrasonic probe in Fig. 2 is used, Fig. 6 is a diagram showing an example of a fan-shaped image displayed when the mechanical scanning ultrasonic probe in Fig. 3 is used, Fig. 7 is a diagram illustrating a display range of an ultrasonic image based on sound ray data of 512 pixels out of sound ray data of 1024 pixels on an entire circumference obtained by the electron scanning ultrasonic probe, Fig. 8 is a schematic diagram of pixel data in a coordinate transformation table and frame memory, illustrating a coordinate transformation process of generating pixel data at pixel locations for display on a monitor screen from sound ray data shown in Fig. 7, Fig. 9 is an enlarged explanatory diagram showing pixels based on any given item of sound ray data in Fig. 8 and pixels at pixel locations for display after coordinate transformation, Fig. 10 is a flowchart showing an example of basic control performed by a control unit in Fig. 1, and Fig. 11 is a flowchart showing an example of control performed by the control unit when the ultrasonic probe in Fig. 1 is pulled out or a range of the ultrasonic probe is changed.

As shown in Fig. 1, the ultrasonic diagnostic apparatus 1 according to the first embodiment includes an electron scanning ultrasonic probe 2, mechanical scanning ultrasonic probe 3, and ultrasonic observation apparatus 4. The ultrasonic observation apparatus 4 is connected with a monitor 5 and operation portion 6.

The electron scanning ultrasonic probe 2 is configured, for example, as an electron scanning ultrasonic endoscope and includes an insertion portion 7 constructed to be slender for ease of insertion, operation portion 8 installed at a rear end of the insertion portion 7, and ultrasonic transducer 9 installed in a distal end portion of the insertion portion 7. The ultrasonic transducer 9 has an array of multiple transducer elements 9a.

The operation portion 8 has an electronic-side connector 10 which is detachably connected to the ultrasonic observation apparatus 4. Although this is not illustrated, the electronic-side connector 10 has an electrical contact connected with a signal line 2a from the ultrasonic transducer 9 and an electronic-side connection detector which detects whether the electron scanning ultrasonic probe 2 is connected to the ultrasonic observation apparatus 4.

In the case of the electron scanning ultrasonic probe 2, the ultrasonic transducer 9 is electrically connected to the ultrasonic observation apparatus 4 via the signal line 2a by the electronic-side connector 10 being connected to the ultrasonic observation apparatus 4.

Incidentally, the electron scanning ultrasonic probe 2 is connected to a light source and video processor (none is shown) when configured, for example, as an electron scanning ultrasonic endoscope. The electron scanning ultrasonic probe 2 has an illumination optical system, objective optical system, and image pickup unit (none is shown) installed in the distal end portion of the insertion portion 7. The electron scanning ultrasonic probe illuminates an inside of a body cavity by means of the illumination optical system using illumination light supplied from the light source, captures reflected light from the illuminated the inside of the body cavity as a subject image by means of objective optical system and, picks up an image by means of the image pickup unit. The electron scanning ultrasonic probe outputs an image pickup signal to the video processor. The video processor generates a standard video signal through signal processing of the image pickup signal and outputs the video signal to an endoscopic image monitor to display an endoscopic image on the endoscopic image monitor.

The mechanical scanning ultrasonic probe 3 has an insertion portion 11 constructed to be slender for ease of insertion and operation portion 12 installed at a rear end of the insertion portion 11. An ultrasonic transducer 14 is fixed to a distal end of a flexible shaft 13 passed through the insertion portion 11 of the mechanical scanning ultrasonic probe 3.

A rear end of the flexible shaft 13 is connected to a rotating drive (not shown) installed in the operation portion 12. The rotating drive rotates the flexible shaft 13 using a motor (not shown), and thereby rotationally drives the ultrasonic transducer 14 in a mechanical fashion. Also, the rotating drive (not shown) has a rotational position detector such as an encoder (not shown) and the like. Space around the ultrasonic transducer 14 is filled with an ultrasonic propagation medium (not shown) which propagates ultrasonic waves.

The operation portion 12 has a mechanical-side connector 15 which is detachably connected to the ultrasonic observation apparatus 4. Although this is not illustrated, the mechanical-side connector 15 has a mechanical-side electrical contact (not shown) connected with a signal line from the rotating drive and a mechanical-side connection detector which detects whether the mechanical scanning ultrasonic probe 3 is connected to the ultrasonic observation apparatus 4.

In the case of the mechanical scanning ultrasonic probe 3, the ultrasonic transducer 14 is electrically connected to the ultrasonic observation apparatus 4 via a signal line passed through the flexible shaft 13 by the mechanical-side connector 15 being connected to the ultrasonic observation apparatus 4.

Incidentally, when configured, for example, as an electron scanning ultrasonic endoscope, the electron scanning ultrasonic probe 2 has a treatment instrument passage channel (not shown). The mechanical scanning ultrasonic probe 3 is passed through the treatment instrument passage channel of the electron scanning ultrasonic endoscope, pushed out of an opening of the treatment instrument passage channel, and thereby inserted in the body cavity.

Next, concrete configuration of the ultrasonic observation apparatus 4 according to the first embodiment will be described with reference to Fig. 1.

The ultrasonic observation apparatus 4 used in the ultrasonic diagnostic apparatus 1 according to the first embodiment is configured to be able to regenerate a coordinate transformation table 23 according to a type or range of the connected ultrasonic probe.

Specifically, as shown in Fig. 1, the ultrasonic observation apparatus 4 has an electronic-side connector receptacle 10a, mechanical-side connector receptacle 15a, electronic-side receiver-transmitter 16, mechanical-side receiver-transmitter 17, signal processing unit 18, and image processing unit 20, control unit 21, all of which are interconnected electrically via a bus 19.

The electronic-side connector 10 of the electron scanning ultrasonic probe 2 is detachably connected to the electronic-side connector receptacle 10a. Consequently, the electron scanning ultrasonic probe 2 is detachably connected to the ultrasonic observation apparatus 4.

Also, the mechanical-side connector 15 of the mechanical scanning ultrasonic probe 3 is detachably connected to the mechanical-side connector receptacle 15a. Consequently, the mechanical scanning ultrasonic probe 3 is detachably connected to the ultrasonic observation apparatus 4.

Incidentally, although this is not illustrated, the electronic-side connector receptacle 10a has a receptacle-side electrical contact which comes into contact with the electrical contact of the electronic-side connector 10 for electrical conduction and has an electronic-side fitting recess into which an electronic-side connection detector protrusion of the electronic-side connector 10 is fitted. On the other hand, although this is not illustrated, the mechanical-side connector receptacle 15a has a receptacle-side electrical contact which comes into contact with the mechanical-side electrical contact of the mechanical-side connector 15 for electrical condition and has a mechanical-side fitting recess into which a mechanical-side connection detector protrusion of the mechanical-side connector 15 is fitted.

The electronic-side and mechanical-side fitting recesses (not shown) are electrically connected to the bus 19 via the signal line. When the electronic-side or mechanical-side connection detector protrusion is fitted into the electronic-side or mechanical-side fitting recess, the control unit 21 electrically connected to the bus 19 conducts and detects that the electronic-side connector 10 or the mechanical-side connector 15 has been connected.

The ultrasonic observation apparatus 4 obtains an echo signal from the connected electron scanning ultrasonic probe 2 or mechanical scanning ultrasonic probe 3, generates an ultrasonic tomographic image based on sound ray data obtained from the echo signal, and displays the ultrasonic tomographic image on the monitor 5.

Specifically, the electronic-side receiver-transmitter 16 transmits ultrasonic pulses to body tissue from the ultrasonic transducer 9 contained in the electron scanning ultrasonic probe 2 connected to the electronic-side connector receptacle 10a and detects the echo signal obtained by receiving the ultrasonic pulses reflected from the body tissue.

Also, the mechanical-side receiver-transmitter 17 transmits ultrasonic pulses to body tissue from the ultrasonic transducer 14 contained in the mechanical scanning ultrasonic probe 3 connected to the mechanical-side connector receptacle 15a and detects the echo signal obtained by receiving the ultrasonic pulses reflected from the body tissue.

The signal processing unit 18 processes echo signals from the electronic-side receiver-transmitter 16 and mechanical-side receiver-transmitter 17.

For example, the signal processing unit 18 generates sound ray data of luminance information according to distances from the ultrasonic transducers 9 and 14 and luminance levels based on the echo signals from the electronic-side receiver-transmitter 16 and mechanical-side receiver-transmitter 17.

The control unit 21 is made up of, for example, a CPU. Using a computing unit 22 and the coordinate transformation table 23, the control unit 21 performs a coordinate transformation of sound ray data generated by the signal processing unit 18 as a result of signal processing. Consequently, the control unit 21 generates pixel data at pixel locations for display on the screen of the monitor 5, and thereby generates one ultrasonic image in a frame memory 24. Subsequently, the control unit 21 outputs pixel data for display (hereinafter referred to as display data) resulting from the ultrasonic images generated on a frame-by-frame basis in the frame memory 24 to the image processing unit 20.

The image processing unit 20 processes and scan-converts the display data received from the control unit 21 and displays the ultrasonic images on the display screen of the monitor 5.

An example of sound ray data generated when the electron scanning ultrasonic probe 2 is connected is shown in Fig. 2.

In this case, the electron scanning ultrasonic probe 2, which supports not only B mode, but also flow mode (Doppler mode), generates sound ray data 25 with a pixel count in a range of 500 to 600 in all directions around the ultrasonic transducer 9, for example, as shown in Fig. 2, each time the mode or range is changed. Schematically, the sound ray data 25 contain multiple items of pixel data 25a.

Even if the range is the same, in a different mode, the electron scanning ultrasonic probe 2 may generate sound ray data 25 with a pixel count of 580, for example, as shown in Fig. 4.

Fig. 5 shows an example of display on the monitor 5 when the sound ray data 25 is generated by the electron scanning ultrasonic probe 2 as shown in Fig. 2.

In this case, the control unit 21 displays an ultrasonic image 2A, for example, of a complete circular shape on the monitor 5 as shown, for example, in Fig. 5, where the ultrasonic image 2A is generated from the sound ray data 25 shown in Fig. 2 through coordinate transformation. Incidentally, since sound ray data 25 is obtained in all directions around the ultrasonic transducer 9 when the electron scanning ultrasonic probe 2 is connected, by processing the sound ray data 25 appropriately, the control unit 21 can display an ultrasonic image 2A of another shape such as a semicircular shape or fan (convex) shape on the monitor 5.

On the other hand, an example of sound ray data generated when the mechanical scanning ultrasonic probe 3 is connected is shown in Fig. 3.

In this case, the mechanical scanning ultrasonic probe 3, which supports two B modes (monochrome), generates sound ray data 26 with a pixel count of 512 or 1024 in a predetermined direction from the ultrasonic transducer 14, for example, as shown in Fig. 3, each time the mode or range is switched. Schematically, the sound ray data 26 contain multiple items of pixel data 26a as in the case of the electron scanning ultrasonic probe 2.

Fig. 6 shows an example of display on the monitor 5 when the sound ray data 26 is generated by the mechanical scanning ultrasonic probe 3 as shown in Fig. 3.

In this case, the control unit 21 displays an ultrasonic image 3A, for example, of a fan shape on the monitor 5 as shown, for example, in Fig. 6, either directly or in magnified form, where the ultrasonic image 3A is generated from the sound ray data 26 shown in Fig. 3 through coordinate transformation.

Next, a configuration of the control unit 21 will be described with reference to Fig. 1 and Figs. 7 to 9, where the control unit 21 is a main component of the ultrasonic observation apparatus 4 according to the first embodiment.

As shown in Fig. 1, the control unit 21 of the ultrasonic observation apparatus 4 includes, the computing unit 22, coordinate transformation table 23, and frame memory 24.

The control unit 21 detects the type or range of the detachable ultrasonic probe and regenerates the coordinate transformation table 23 based on the detected type or range of the detachable ultrasonic probe using the computing unit 22 and frame memory 24.

If, for example, the pixel count of the sound ray data supplied by the signal processing unit 18 differs from the number of pixel locations for display on the screen of the monitor 5, the computing unit 22 performs a computing process to interpolate or skip pixels in the sound ray data so that the pixel count of the sound ray data will match the number of pixel locations for display. Then, the control unit 21 regenerates the coordinate transformation table 23 based on computational results produced by the computing unit 22.

Incidentally, if the pixel count of the inputted sound ray count matches the number of pixel locations for display, there is no need to regenerate the coordinate transformation table 23 for use to generate the display data at the pixel locations for display.

After coordinate transformation of the sound ray data is performed using the coordinate transformation table 23 regenerated based on the computational results produced by the computing unit 22, the frame memory 24 stores the resulting display data on a frame-by-frame basis under the control of the control unit 21. Under the control of the control unit 21, the frame-by-frame display data is processed by the image processing unit 20, and consequently an ultrasonic image is displayed on the monitor 5.

The control unit 21 controls that a regeneration process of the coordinate transformation table 23 via the computing process of the computing unit 22 will be performed when the electron scanning ultrasonic probe 2 or mechanical scanning ultrasonic probe 3 is replaced, when the range is changed at a command from the operation portion 6, or when the mode is switched between B mode and flow mode at a command from the operation portion 6 if the electron scanning ultrasonic probe 2 is in operation.

Now, the regeneration process of the coordinate transformation table 23 performed by the control unit 21 will be described in more detail with reference to Figs. 7 to 9.

For example, if the pixel count of the sound ray data 25 obtained by the electron scanning ultrasonic probe 2 is 512 as shown in Fig. 7, since the sound ray data 25 is obtained in all the directions around the ultrasonic transducer 9, a 1024-pixel ultrasonic image 2A of a complete circular shape is displayed under the control of the control unit 21.

Suppose, it is detected that the electron scanning ultrasonic probe 2 is pulled out (replaced) or that the range is changed in the operation portion 6.

It is assumed that the pixel count of sound ray data obtained from the newly connected electron scanning ultrasonic probe 2 or based on the changed range is 580 as shown in Fig. 4 and that a display area 30 (a quadrant with a radius of L) shown in Fig. 7 is specified.

In this case, the control unit 21 cannot perform coordinate transformation using the coordinate transformation table 23 existing before the replacement or range changing of the electron scanning ultrasonic probe 2. Thus, the control unit 21 detects the type or range of the connected electron scanning ultrasonic probe based on inputted sound ray data (see, for example, Fig. 4) or an operation signal from the operation portion 6 and makes the computing unit 22 perform a computing process for interpolation based on results of the detection to bring the inputted sound ray data into correspondence with the pixel locations for display on the screen of the monitor 5.

Based on computational results produced by the computing unit 22, the control unit 21 regenerates the coordinate transformation table 23. Here, the pixel locations for display in the frame memory 24 match the pixel locations for display on the screen of the monitor 5.

Fig. 8 schematically shows the pixel locations for display in the display area 30 shown in Fig. 7, where information about the pixel locations for display is stored in the coordinate transformation table 23 and frame memory 24. That is, the coordinate transformation table 23 prepared before the regeneration process cannot be used for current coordinate transformation because in terms of locations, the pixel data 25a in the sound ray data 25 does not match the pixel data 24a for display on the screen of the monitor 5.

Therefore, the control unit 21 makes the computing unit 22 perform a computing process for interpolation using the pixel data 25a from the inputted sound ray data 25 to obtain pixel data 24a at pixel locations for display on the screen of the monitor 5, for example, as shown in Fig. 9.

Then, based on computational results, the coordinate transformation table 23 can be regenerated under the control of the control unit 21. The regenerated coordinate transformation table 23 can be used for coordinate transformation to prepare the pixel data for display on the screen of the monitor 5.

It has been stated above that the coordinate transformation table 23 is regenerated after the electron scanning ultrasonic probe 2 is pulled out (replaced) or the range of the electron scanning ultrasonic probe 2 is changed. However, since the coordinate transformation table 23 can also be regenerated to accommodate sound ray data with different pixel counts, the coordinate transformation table 23 can, of course, be regenerated after the mechanical scanning ultrasonic probe 3 which does not determine the pixel count of available pixel data is pulled out (replaced) or its range is changed, as in the case of the electron scanning ultrasonic probe 2.

Incidentally, if both the electron scanning ultrasonic probe 2 and mechanical scanning ultrasonic probe 3 are connected, at a command from the operation portion 6, the control unit 21 can cause one of the two probes to operate.

The control unit 21 has a data combining unit (not shown) and can control to make the data combining unit combine, as required, the sound ray data obtained by the electron scanning ultrasonic probe 2 and mechanical scanning ultrasonic probe 3.

Transmission frequencies of the B mode and the flow mode are set to predetermined sending frequencies, but the control unit 21 can control to make the electronic-side receiver-transmitter 16 and mechanical-side receiver-transmitter 17 change the transmission frequencies of the B mode and flow mode separately.

When the electron scanning ultrasonic probe 2 is connected, the control unit 21 can make the image processing unit 20 generate display data of three-dimensional ultrasonic images using obtained sound ray data or generate display data to display patient information and other medical information inputted from the operation portion 6 on screen as ultrasonic images. Of course the control unit 21 can perform display control of display data and the like of three-dimensional ultrasonic images. A technique related to such three-dimensional ultrasonic image display will be described later.

Next, operation of the ultrasonic observation apparatus 4 according to the first embodiment will be described with reference to Figs. 10 and 11.

Suppose a surgeon turns on the ultrasonic observation apparatus 4 used in the ultrasonic diagnostic apparatus 1 shown in Fig. 1. In response, the control unit 21 reads a program shown in Fig. 10 out of memory (not shown) and executes the program.

That is, in Step S1, the control unit 21 controls to make the electronic-side receiver-transmitter 16 or mechanical-side receiver-transmitter 17 transmit ultrasonic pulses to body tissue from the ultrasonic transducer 9 or 14 contained in the connected electron scanning ultrasonic probe 2 or mechanical scanning ultrasonic probe 3 and detects the echo signal obtained by receiving the ultrasonic pulses reflected from the body tissue.

Then, in Step S2, the control unit 21 makes the signal processing unit 18 generate sound ray data of luminance information according to the distance from the ultrasonic transducer 9 or 14 and luminance level based on the echo signal from the electronic-side receiver-transmitter 16 or mechanical-side receiver-transmitter 17.

Then, in Step S3, the control unit 21 performs coordinate transformation using the computing unit 22 and coordinate transformation table 23 to convert the sound ray data processed by the signal processing unit 18 into pixel data at pixel locations for display on the screen of the monitor 5.

Then, in Step S4, the control unit 21 generates one ultrasonic image in the frame memory 24 as a result of the coordinate transformation. Subsequently, the control unit 21 outputs pixel data for display (hereinafter referred to as display data) resulting from the ultrasonic images generated on a frame-by-frame basis in the frame memory 24 to the image processing unit 20. Consequently, the image processing unit 20 processes and scan-converts the display data received from the control unit 21 and displays the ultrasonic images on the display screen of the monitor 5.

Suppose, for example, the surgeon pulls out (replaces) the electron scanning ultrasonic probe 2 or mechanical scanning ultrasonic probe 3 or changes the range of either probe, or switches between B mode and flow mode if the electron scanning ultrasonic probe 2 is connected.

Here, as shown in Fig. 11, the program read out of memory (not shown) is constantly running. Consequently, in Step S10, the control unit 21 detects that the electron scanning ultrasonic probe 2 or mechanical scanning ultrasonic probe 3 has been pulled out (replaced) or that the range of either probe has been switched, or that the mode has been switched between B mode and flow mode if the electron scanning ultrasonic probe 2 is connected.

The control unit 21 can detect if the electron scanning ultrasonic probe 2 or mechanical scanning ultrasonic probe 3 has been pulled out (replaced) because when the electronic-side or mechanical-side connection detector protrusion is fitted into the electronic-side or mechanical-side fitting recess, conduction occurs allowing the control unit 21 to detect that the electronic-side connector 10 or mechanical-side connector 15 has been connected. Use of this mechanism makes it possible to automatically enable an ultrasonic probe connector upon connection if no other connector is connected. Also, the range changing can be detected through recognition of an operation signal from the operation portion 6. Also, the switching between B mode and flow mode may be detected through recognition of the pixel count of inputted sound ray data.

In such a case, however, the control unit 21 cannot perform coordinate transformation using the coordinate transformation table 23 prepared before the replacement or range changing of the electron scanning ultrasonic probe 2 or mechanical scanning ultrasonic probe 3 or before the switching between B mode and flow mode.

Thus, in Step S11, the control unit 21 makes the computing unit 22 perform a computing process for interpolation to convert the newly obtained sound ray data into pixel data at pixel locations for display on the screen of the monitor 5, where the newly obtained sound ray data is the sound ray data obtained using the newly connected ultrasonic probe (electron scanning ultrasonic probe 2 or mechanical scanning ultrasonic probe 3), the changed range, or the mode (B mode or flow mode) switched to.

Based on computational results produced by the computing unit 22, the control unit 21 regenerates the coordinate transformation table 23. That is, the resulting coordinate transformation table 23 allows the control unit 21 to perform coordinate transformation and thereby obtain pixel data at pixel locations for display on the screen of the monitor 5.

This makes it possible to display optimum ultrasonic images on the monitor 5 even after removal (replacement) or range changing of the electron scanning ultrasonic probe 2 or mechanical scanning ultrasonic probe 3 or switching between B mode and flow mode.

Thus, according to the first embodiment, since the coordinate transformation table 23 can be regenerated to accommodate changes in the type or range of the ultrasonic probe, it is possible to generate and display optimum ultrasonic images even when the type or range of the ultrasonic probe is changed. This reduces memory requirements and costs. The ultrasonic observation apparatus 4 can be connected with both the electron scanning ultrasonic probe 2 and mechanical scanning ultrasonic probe 3.

As described earlier, when connected with the electron scanning ultrasonic probe 2, the ultrasonic observation apparatus 4 used in the ultrasonic diagnostic apparatus 1 according to the present invention can perform display control of display data and the like of three-dimensional ultrasonic images by making the image processing unit 20 generate display data of three-dimensional ultrasonic images using obtained sound ray data or generate display data to display patient information and other medical information inputted from the operation portion 6 on screen as ultrasonic images.

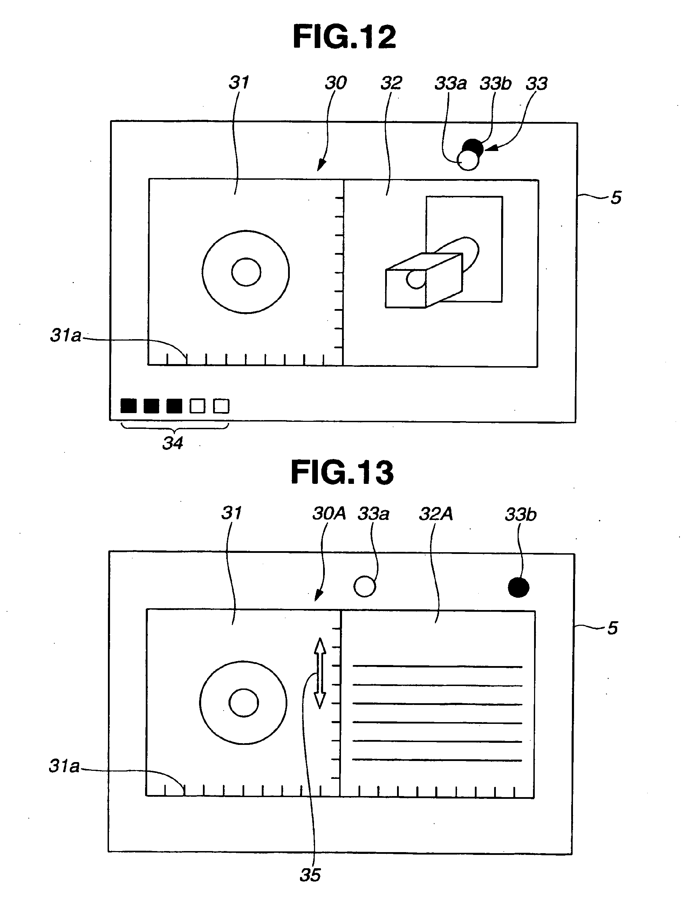

A technique related to such three-dimensional ultrasonic image display in the ultrasonic observation apparatus 4 and other relevant techniques will be described with reference to Figs. 12 to 21.

When operating in three-dimensional ultrasonic image display mode to display a three-dimensional ultrasonic image as described above, the control unit 21 of the ultrasonic observation apparatus 4 can also generate and display slice images (horizontal linear slice images) taken from the three-dimensional ultrasonic image horizontally parallel to an insertion axis of the ultrasonic transducer 9. Of course, the control unit 21 may generate and display slice images taken from the three-dimensional ultrasonic image vertically with respect to the insertion axis of the ultrasonic transducer 9.

Incidentally, horizontal linear slice images are taken at predetermined slice intervals, which are, for example, five in number. The slice intervals are specified with a trackball or the like of the operation portion 6. Also, the capabilities described in relation to the electron scanning ultrasonic probe 2 are also available when the mechanical scanning ultrasonic probe 3 is connected.

Also, the control unit 21 performs more slowly in the three-dimensional ultrasonic image display mode than in normal ultrasonic image display mode. To deal with this, the control unit 21 generates and displays a relatively low-definition three-dimensional ultrasonic image when a three-dimensional ultrasonic image is being displayed live, but generates and displays a high-definition three-dimensional ultrasonic image if the surgeon gives a freeze command from the operation portion 6, freezing the live display. This makes it possible to reduce loads produced by the control unit 21 in generating three-dimensional ultrasonic images.

The ultrasonic observation apparatus 4 has a manual scanning mode which allows the surgeon to generate a three-dimensional ultrasonic image by operating the electron scanning ultrasonic probe 2 manually.

The ultrasonic observation apparatus 4 is capable of dual-screen display of a three-dimensional ultrasonic image and two-dimensional ultrasonic image. Examples of such dual-screen display are shown in Figs. 12 and 13.

As shown in Fig. 12, when the electron scanning ultrasonic probe 2 is connected, the control unit 21 of the ultrasonic observation apparatus 4 can control to make the image processing unit 20 present a dual-screen display section 30 on the screen of the monitor 5, displaying, for example, on left and right, a first screen 31 which can display a normal two-dimensional ultrasonic image (radial ultrasonic image) and a second screen 32 which can display a three-dimensional ultrasonic image.

In that case, a scale 31 a for use to recognize size of the ultrasonic image is displayed by the control unit 21 at least on one side of a first screen 31 on which the two-dimensional ultrasonic image is displayed. This allows the surgeon to estimate actual size of an observed site by comparing the size of the two-dimensional ultrasonic image with the scale 31 a.

Also, when a three-dimensional ultrasonic image is displayed, it may be difficult to recognize depth direction of the electron scanning ultrasonic probe 2, i.e., to distinguish between a distal end and rear end of the electron scanning ultrasonic probe 2, on the second screen on which the three-dimensional ultrasonic image is displayed. Therefore, the control unit 21 displays marks 33 on top of the second screen 32 of the dual-screen display section 30 to distinguish between the distal end and rear end of the electron scanning ultrasonic probe 2.

The marks 33 include, for example, a white mark 33a in the form of ○ which indicates the distal end of the ultrasonic probe and black mark 33b in the form of ● which indicates the rear end of the ultrasonic probe. The white mark 33a and black mark 33b may be overlapped as shown in Fig. 12. In that case, by looking at the degree of overlap between the white mark 33a and black mark 33b, it is possible to tell at once which of the distal and rear ends of the ultrasonic probe is displayed on the near side and which is displayed on the far side.

The ultrasonic observation apparatus 4 has a capability to store images in digital format. There is demand for information about remaining HDD space for image storage because it is desired to know how many more images can be stored.

Thus, the control unit 21 displays a bar indicator 34, for example, at bottom of the dual-screen display section 30 to indicate the remaining space on the HDD including the control unit 21. Incidentally, the bar indicator 34 may be substituted with a graphical indicator or any other item which indicates the remaining space on the HDD including the control unit 21.

As shown in Fig. 13, when displaying the dual-screen display section 30A, the control unit 21 may display successively on the second screen 32A ultrasonic images of cross sections according to cursor position by moving a cursor 35 provided near the scale 31 a on the first screen 31.

In this case, when the surgeon presses the cursor 35, for example, for a long time by manipulating the operation portion 6, the control unit 21 automatically displays successive ultrasonic images of cross sections taken at predetermined intervals, based on a preset direction of the cursor 35.

Incidentally, although the cursor 35 is provided vertically along the first screen 31, this is not restrictive. The cursor 35 may be provided horizontally along the first screen 31 and ultrasonic images of cross sections taken at predetermined horizontal intervals may be displayed successively. Besides, the white mark 33a and black mark 33b are displayed at top of the second screen 32A to distinguish between the distal end and rear end of the ultrasonic probe. This allows the surgeon to distinguish between the distal end and rear end of the ultrasonic probe on the second screen 32A, making it easier to analyze the ultrasonic images of cross sections displayed on the second screen 32A.

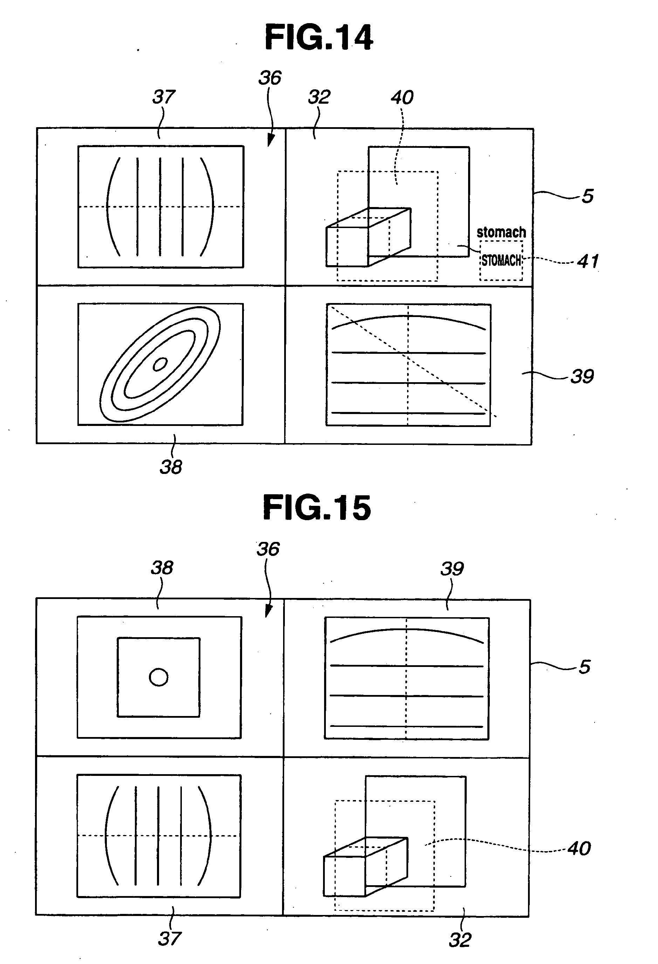

Fig. 14 is a diagram showing an example of display created by the ultrasonic observation apparatus in three-dimensional ultrasonic image display mode. Fig. 15 is a diagram showing a variation of the three-dimensional ultrasonic image display mode in Fig. 14.

As shown in Fig. 14, when operating in the three-dimensional ultrasonic image display mode to display a three-dimensional ultrasonic image, the control unit 21 of the ultrasonic observation apparatus 4 can control to make the image processing unit 20 present a quad-screen display section 36 on the screen of the monitor 5, displaying a first screen 37, second screen 38, third screen 39, and fourth screen 32 which can display mutually different ultrasonic images.

In this case, the quad-screen display section 36 includes, for example, the first screen 37 placed in upper left part, second screen 38 placed in lower left part, third screen 39 placed in lower right part, and fourth screen 32 placed in upper right part.

The first screen 37 displays slice images of the ultrasonic image displayed on the fourth screen 32 (described later), taken vertically with respect to the insertion axis of the ultrasonic transducer 9. The second screen 38 displays a normal two-dimensional ultrasonic image. The third screen 39 displays slice images of the ultrasonic image displayed on the fourth screen 32 (described later) taken horizontally parallel to the insertion axis of the ultrasonic transducer 9.

According to this example, the control unit 21 displays a reference cross section 40 serving as specifying means used to obtain a three-dimensional slice image (sectional image) in a desired direction from a three-dimensional ultrasonic image displayed on the fourth screen 32.

The desired direction specified using the reference cross section 40 may be, for example, any of three directions on the three-dimensional ultrasonic image: horizontal, vertical, or diagonal with respect to the insertion axis of the ultrasonic transducer 9. Of course, the direction which can be specified is not limited to the three directions, and any direction may be specified by moving the reference cross section 40 freely.

Incidentally, the desired direction specified using the reference cross section 40 may be indicated, for example, with a trackball, a keyboard, or arrow keys of the operation portion 6.

In the display example shown in Fig. 14, a slice position vertical with respect to the insertion axis of the ultrasonic transducer 9 is specified using the reference cross section 40 and a three-dimensional slice image at the slice position is displayed on the third screen 39.

Since the control unit 21 presents the quad-screen display section 36 with the reference cross section 40 allowing a three-dimensional slice image in a desired direction to be displayed, it is possible to analyze the three-dimensional ultrasonic image in more detail.

Incidentally, the control unit 21 may control to cause comments 41 such as information about body tissue and the like to be displayed over the three-dimensional ultrasonic image displayed on the fourth screen 32.

Also, as illustrated in the variation in Fig. 15, the control unit 21 may change layout of the quad-screen display section 36 by arranging the first screen 37, second screen 38, third screen 39, and fourth screen 32 on the screen of the monitor 5 in an easy to see fashion.

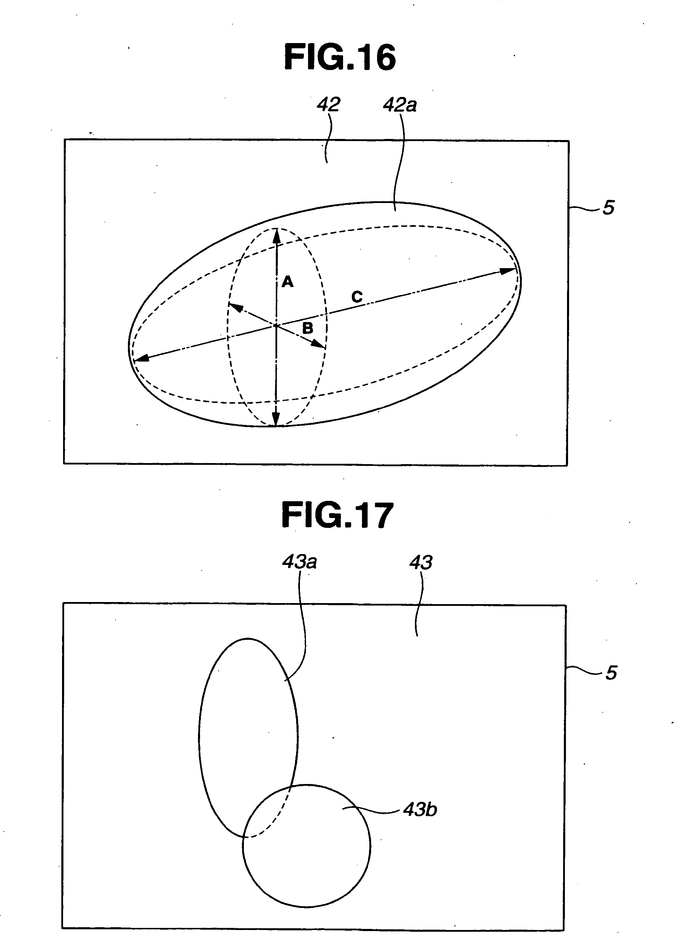

Figs. 16 and 17 illustrate a capability to measure a volume of an observed site on a three-dimensional ultrasonic image in three-dimensional ultrasonic image display mode, where Fig. 16 is a diagram showing a display example of how to measure the volume by specifying three points at the observed site on the displayed three-dimensional ultrasonic image and Fig. 17 is a diagram showing a display example of how volume measurements are allowed to be taken twice at the observed site on the three-dimensional ultrasonic image.

In the three-dimensional ultrasonic image display mode, the ultrasonic observation apparatus 4 can measure the volume of an observed site on a displayed three-dimensional ultrasonic image using available two-dimensional sound ray data.

In that case, the control unit 21 displays a three-dimensional ultrasonic image 42, for example, on the monitor 5 and allows the surgeon to enter distance data which represents at least three dimensions (e.g., vertical distance A, lateral distance B, and depthwise distance C) of an observed site 42a on the three-dimensional ultrasonic image 42.

The surgeon enters appropriate distance data using the keyboard of the operation portion 6.

The control unit 21 makes the computing unit 22 determine the volume of the observed site 42a on the displayed three-dimensional ultrasonic image 42 through computations based on the entered three types of distance data, and displays the volume, for example, over the ultrasonic image 42. This makes it possible to estimate size of the observed site on the three-dimensional ultrasonic image.

According to this example, the volume of the observed site on the three-dimensional ultrasonic image can be measured twice, for example, as shown in Fig. 17. Furthermore, the present invention may be configured to measure the volume more than twice if necessary. When multiple measurements of the observed site are taken, the control unit 21 may display observed sites 43a and 43b in different colors.

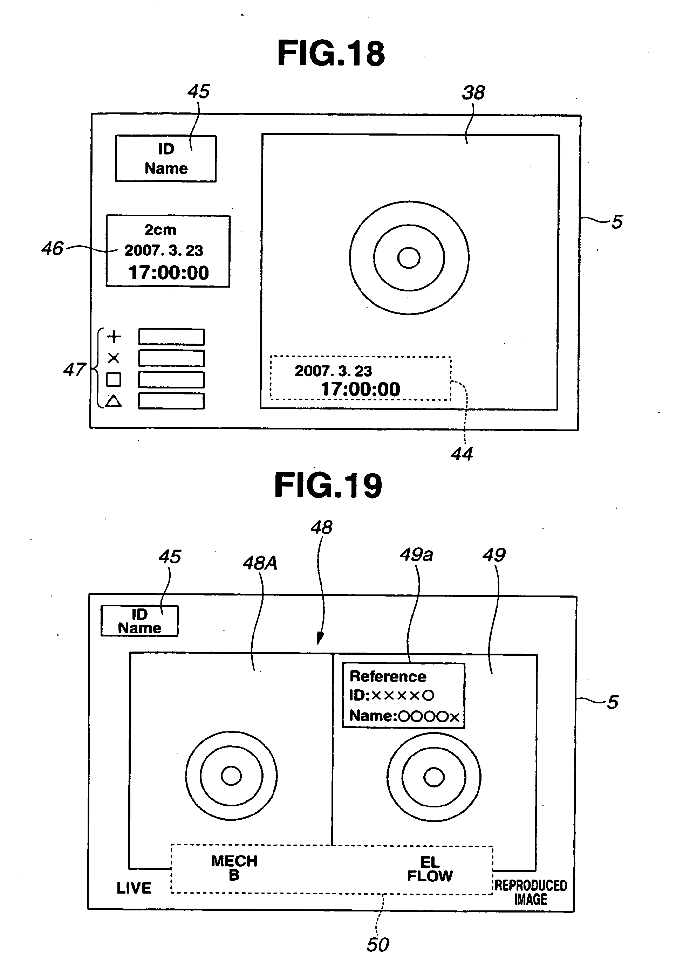

Incidentally, the ultrasonic observation apparatus 4 used in the ultrasonic diagnostic apparatus 1 according to the present invention can perform functions described below when displaying normal two-dimensional ultrasonic images. The functions will be described with reference to Figs. 18 to 21.

Fig. 18 is a diagram showing a display example of a normal two-dimensional ultrasonic image on a display screen.

As shown in Fig. 18, in normal two-dimensional ultrasonic image display mode, if, for example, the surgeon gives a freeze command from the operation portion 6, the control unit 21 of the ultrasonic observation apparatus 4 displays a freeze time indicator 44 over an ultrasonic image 38 on the monitor 5, indicating that a freeze mode is on.

The freeze time indicator 44 provides, for example, time information about the time when the image was frozen. The freeze time indicator 44 is displayed at a position where it will not overlap the frozen ultrasonic image 38.

Incidentally, in the freeze mode in which the freeze time indicator 44 is displayed, the control unit 21 controls to make a time display unit 46 update current time as required.

Fig. 19 is a diagram showing a variation of dual-screen display mode of the ultrasonic observation apparatus. In the dual-screen display mode described above, the dual-screen display section 30 is displayed, presenting a normal two-dimensional ultrasonic image and three-dimensional ultrasonic image. In this example, as shown in Fig. 19, a dual-screen display section 48 is displayed, presenting a normal live ultrasonic image and an image reproduced by being read out of memory (not shown) in the control unit 21.

That is, as shown in Fig. 19, the control unit 21 of the ultrasonic observation apparatus 4 makes the image processing unit 20 present a dual-screen display section 48 on the screen of the monitor 5, displaying, for example, on left and right, a first screen 48A which can display a normal two-dimensional ultrasonic image (radial ultrasonic image) and a second screen 49 which can display an image reproduced (reproduced ultrasonic image) by being read out of memory (not shown) in the control unit 21.

In this case, the control unit 21 displays an identification information display section 49a over the reproduced image displayed on the second screen 49 to identify the reproduced image from live ultrasonic images. The identification information display section 49a contains patient information such as a patient number or patient name. Incidentally, the identification information display section 49a may be hidden selectively whenever unnecessary.

The control unit 21 displays a patient information display section 45 in an upper proximal end portion of the dual-screen display section 30 to display a patient number and patient name. The display of the patient information display section 45 can be suppressed at a command from the surgeon as required (e.g., the patient number can be erased when a button is pressed and the name can be erased when the button is pressed next).

Also, according to this example, in the dual-screen display mode, the control unit 21 can display a selection menu 50 for use to select modes of ultrasonic images to be displayed on the first screen 48A and second screen 49. That is, when the surgeon gives a command by manipulating the operation portion 6, the control unit 21 displays the selection menu 50 on the dual-screen display section 48.

The selection menu 50 allows the surgeon to select among live mode, mechanical scanning mode by the mechanical scanning ultrasonic probe 3, and B mode on the first screen 48A and to select among reproduction mode, electron scanning mode by the electron scanning ultrasonic probe 2, and flow mode on the second screen 49.

Consequently, the dual-screen display section 48 can display ultrasonic images in different modes simultaneously, making it possible to analyze the ultrasonic images efficiently.

Incidentally, the dual-screen display mode described above is not limited to normal two-dimensional ultrasonic image display, and is applicable to the three-dimensional ultrasonic image display. Also, the ultrasonic observation apparatus 4 according to this example allows the surgeon to display a menu for various settings of the entire system by manipulating the operation portion 6 although this is not illustrated.

In that case, the menu contains an item for setting a display language used for the ultrasonic observation apparatus, allowing the display language to be switched without restarting the control unit 21 of the ultrasonic observation apparatus 4.

Also, when the control unit 21 is displaying the menu, operating instructions or other comments may be displayed, for example, at bottom of a display screen of the menu. Also, the control unit 21 can display a gray scale for monitor adjustment in part of the screen of the monitor 5.

The present apparatus has picture-in-picture (PinP) capabilities. However, when a menu such as described above is displayed, any child window of PinP format being displayed is turned off automatically to avoid obstructing menu operations.

If the surgeon makes an operation mistake when manipulating the operation portion 6 by watching the display screen of the menu or the like, the control unit 21 displays an error message accordingly, and then displays operating instructions or other comments on screen. This makes it possible to show correct operating procedures to the surgeon.

The control unit 21 can clear ultrasonic images and other displays such as comments and menus on the monitor 5 after a predetermined time.

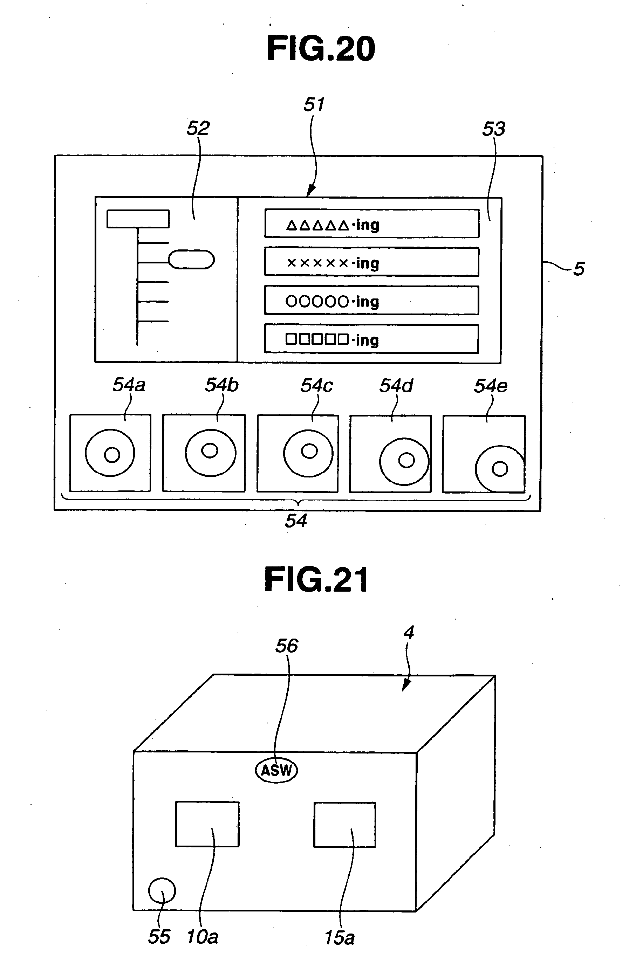

Fig. 20 is a diagram showing an example of display on a display screen to illustrate capabilities to display thumbnails by reading out prestored ultrasonic images.

As shown in Fig. 20, the control unit 21 of the ultrasonic observation apparatus 4 can display thumbnails of ultrasonic images read out of memory (not shown).

Specifically, the control unit 21 can display a dual-screen display section 51 and thumbnail display section 54, the dual-screen display section 51 displaying, for example, on left and right, a first screen 52 which displays a tree structure of folders and the like stored in memory (not shown) and a second screen 53 which displays file numbers in the folders displayed on the first screen 52 and the thumbnail display section 54 displaying thumbnails of reproduced ultrasonic images whose file numbers are displayed on the second screen 53.

That is, the control unit 21 successively displays reproduced ultrasonic images whose file numbers are displayed on the second screen 53, in thumbnail display locations 54a to 55e of the thumbnail display section 54. This makes it possible to analyze in detail changes in the observed site based on the reproduced ultrasonic images.

When replacing the ultrasonic probe of a conventional ultrasonic observation apparatus, the surgeon turns off the ultrasonic observation apparatus completely by pressing a power switch. After the replacement of the ultrasonic probe, the surgeon turns on the ultrasonic observation apparatus by pressing the power switch again. However, this method takes time to start up the apparatus.

To deal with this, in the ultrasonic observation apparatus 4 used in the ultrasonic diagnostic apparatus 1 according to the present invention, an active switch 56 is installed, for example, on a front panel as shown in Fig. 21 to supply power to the electronic-side connector receptacle 10a and mechanical-side connector receptacle 15a connected with the electron scanning ultrasonic probe 2 and mechanical scanning ultrasonic probe 3.

This makes it possible to turn off power supply to the electronic-side connector receptacle 10a and mechanical-side connector receptacle 15a before replacement of the ultrasonic probe by simply pressing the active switch 56 instead of turning off power by pressing a power switch 55.

After the replacement of the ultrasonic probe, by pressing the active switch 56 again, it is possible to turn on the power supply to the electronic-side connector receptacle 10a and mechanical-side connector receptacle 15a, and thereby make the ultrasonic observation apparatus 4 ready for operation quickly. Also, the installation of the active switch 56 makes it possible to replace the ultrasonic probe with a sense of security.

The active switch 56 is electrically connected to the control unit 21, and the control unit 21 controls power supply to the electronic-side receiver-transmitter 16 and mechanical-side receiver-transmitter 17 based on switching operation of the active switch 56.

Also, when the active switch 56 is off, the surgeon may forget to turn on the active switch 56 after replacement of the ultrasonic probe or in freeze mode.

Thus, according to this example, after it is confirmed that an ultrasonic probe is mounted or if a freeze button is off, the control unit 21 can turn on the active switch 56 automatically after a predetermined time. This makes it possible to carry out an ultrasonic inspection quickly by turning on the active switch 56 even if the surgeon forgets to do so.

Incidentally, the ultrasonic observation apparatus 4 according to this example can record ultrasonic images in the memory (not shown) in the control unit 21.

In this case, an LED is installed on the front panel of the ultrasonic observation apparatus 4, and the control unit 21 can inform the surgeon that ultrasonic image data is being written, by lighting or flashing the LED when storing the ultrasonic image data in the memory (not shown) in the control unit 21 although this is not illustrated.

Improvements have been made to the ultrasonic observation apparatus 4 according to this example to optimally display ultrasonic images from a new ultrasonic probe after replacement.

Specifically, an amount of off-centeredness has been determined according to the type of ultrasonic probe, and the surgeon sets the amount of off-centeredness for the newly replaced ultrasonic probe, for example, using a menu. An ultrasonic probe with a large ultrasonic transducer is displaced from a rotation axis when ultrasonic transducer rotates or vibrates. The amount of off-centeredness is data used to correct for such displacement.

Thus, by making the signal processing unit 18 generate sound ray data based on the set amount of off-centeredness of the ultrasonic probe, the control unit 21 can generate and display optimum ultrasonic images using the newly replaced ultrasonic probe.

Incidentally, data on the amount of off-centeredness for each type of ultrasonic probe may be prestored in the memory (not shown) in the control unit 21. Then, the control unit 21 can detect the type of the connected ultrasonic probe and read and use appropriate data on the amount of off-centeredness.

Also, when generating an image on the ultrasonic observation apparatus 4, the surgeon is allowed to adjust brightness (luminance) of the image according to distance. The ultrasonic observation apparatus 4 simplifies the setting of the adjustment by allowing the surgeon to specify only luminance at the closest position to the ultrasonic transducer and luminance at the most distant position on the screen and interpolating luminance at intermediate positions.

## Claims

1. An ultrasonic observation apparatus (4) being adapted to generate an ultrasonic image from sound ray data obtained by a detachable ultrasonic probe (2, 3), comprising:
a control unit (21) being adapted to detect a type or range of the detachable ultrasonic probe (2, 3),
wherein the control unit (21) includes a computing unit (22), a coordinate transformation table (23), and a frame memory (24), the coordinate transformation table (23) being adapted to generate image data at pixel locations for display from the sound ray data obtained by the ultrasonic probe (2, 3),
**characterized in that**
the computing unit (22) being adapted to perform a computing process comprising interpolation of the sound ray data based on the detected type or range of the detachable ultrasonic probe (2, 3) to bring the sound ray data into correspondence with the pixel locations for display on a display unit (5), wherein
the control unit (21) is adapted to regenerate the coordinate transformation table (23) based on the result of the computing process performed by the computing unit (22).

2. The ultrasonic observation apparatus (4) according to claim 1, wherein the ultrasonic observation apparatus (4) is connected with at least one of an electron scanning ultrasonic probe (2) and a mechanical scanning ultrasonic probe (3) as the detachable ultrasonic probe.

3. The ultrasonic observation apparatus (4) according to claim 1, wherein the control unit (21) being adapted to regenerate the coordinate transformation table (23) upon replacement of the ultrasonic probe (2, 3).

4. The ultrasonic observation apparatus (4) according to claim 1, wherein the control unit (21) being adapted to regenerate the coordinate transformation table (23) upon a change in the range.

5. The ultrasonic observation apparatus (4) according to claim 1, wherein the control unit (21) being adapted to detect switching between B mode and flow mode and to regenerate the coordinate transformation table (23) upon detection of the switching between the B mode and the flow mode.

## Patentansprüche

1. Ultraschallbeobachtungsvorrichtung (4), die dazu eingerichtet ist, aus von einer abnehmbaren Ultraschallsonde (2, 3) erhaltenen Schallwellendaten ein Ultraschallbild zu erzeugen, aufweisend:
eine Steuerungseinheit (21), die dazu eingerichtet ist, eine Art oder einen Bereich der abnehmbaren Ultraschallsonde (2, 3) zu erfassen,
wobei die Steuerungseinheit (21) eine Recheneinheit (22), eine Koordinatentransformationstabelle (23) und einen Bildspeicher (24) enthält, und die Koordinatentransformationstabelle (23) dazu eingerichtet ist, Bilddaten an Pixelpositionen zu erzeugen, um die durch die Ultraschallsonde (2, 3) erhaltenen Schallwellendaten anzuzeigen,
**dadurch gekennzeichnet, dass**
die Recheneinheit (22) dazu eingerichtet ist, einen Rechenprozess auszuführen, der Interpolation der Schallwellendaten basierend auf der erfassten Art oder dem erfassten Bereich der abnehmbaren Ultraschallsonde (2, 3) aufweist, um die Schallwellendaten zum Anzeigen auf einer Anzeigeeinheit (5) in Übereinstimmung mit den Pixelpositionen zu bringen, wobei
die Steuerungseinheit (21) dazu eingerichtet ist, die Koordinatentransformationstabelle (23) basierend auf dem Ergebnis des durch die Recheneinheit (22) ausgeführten Rechenprozesses zu regenerieren.

2. Ultraschallbeobachtungsvorrichtung (4) nach Anspruch 1, bei der die Ultraschallbeobachtungsvorrichtung (4) verbunden ist mit mindestens einem einer Elektronenabtast-Ultraschallsonde (2) und einer mechanischen Abtast-Ultraschallsonde (3) als die abnehmbare Ultraschallsonde.

3. Ultraschallbeobachtungsvorrichtung (4) nach Anspruch 1, bei der die Steuerungsvorrichtung (21) dazu eingerichtet ist, bei Ersetzung der Ultraschallsonde (2, 3) die Koordinatentransformationstabelle (23) zu regenerieren.

4. Ultraschallbeobachtungsvorrichtung (4) nach Anspruch 1, bei dem die Steuerungseinheit (21) dazu eingerichtet ist, bei einer Änderung des Bereichs die Koordinatentransformationstabelle (23) zu regenerieren.

5. Ultraschallbeobachtungsvorrichtung (4) nach Anspruch 1, bei der die Steuerungseinheit (21) dazu eingerichtet ist, ein Schalten zwischen einem B-Modus und einem Fluss-Modus zu erfassen und bei Erfassung des Schaltens zwischen dem B-Modus und dem Fluss-Modus die Koordinatentransformationstabelle (23) zu regenerieren.

## Revendications

1. Appareil d'observation ultrasonique (4) conçu pour engendrer une image ultrasonique à partir de données de rayon sonore obtenues par une sonde ultrasonique amovible (2, 3), comprenant :
une unité de commande (21) conçue pour détecter un type ou une portée de la sonde ultrasonique amovible (2, 3),
dans lequel l'unité de commande (21) comprend une unité de calcul (22), une table de transformation de coordonnées (23) et une mémoire d'image (24), la table de transformation de coordonnées (23) étant conçue pour engendrer des données d'image à des positions de pixels à afficher, à partir des données de rayon sonore obtenues par la sonde ultrasonique (2, 3),
**caractérisé en ce que** :
l'unité de calcul (22) est conçue pour exécuter un processus de calcul comprenant une interpolation des données de rayon sonore sur la base du type détecté ou de la portée détectée de la sonde ultrasonique amovible (2, 3) afin de mettre les données de rayon sonore en correspondance avec les positions de pixels à afficher sur une unité d'affichage (5), dans lequel
l'unité de commande (21) est conçue pour régénérer la table de transformation de coordonnées (23) sur la base du résultat du processus de calcul exécuté par l'unité de calcul (22).

2. Appareil d'observation ultrasonique (4) selon la revendication 1, dans lequel l'appareil d'observation ultrasonique (4) est connecté au moins à une sonde ultrasonique à balayage électronique (2) ou à une sonde ultrasonique à balayage mécanique (3) en tant que sonde ultrasonique amovible.

3. Appareil d'observation ultrasonique (4) selon la revendication 1, dans lequel l'unité de commande (21) est conçue pour régénérer la table de transformation de coordonnées (23) lors du remplacement de la sonde ultrasonique (2, 3).

4. Appareil d'observation ultrasonique (4) selon la revendication 1, dans lequel l'unité de commande (21) est conçue pour régénérer la table de transformation de coordonnées (23) lors d'un changement de la portée.

5. Appareil d'observation ultrasonique (4) selon la revendication 1, dans lequel l'unité de commande (21) est conçue pour détecter une commutation entre le mode B et le mode Doppler et pour régénérer la table de transformation de coordonnées (23) lors de la détection de la commutation entre le mode B et le mode Doppler.
